# EUROPEAN PATENT SPECIFICATION

(11) **EP 2 018 830 B1**
(45) Date of publication and mention of the grant of the patent: **21.09.2011**
(21) Application number: 08013445.5
(22) Date of filing: 25.07.2008
(51) Int. Cl.: A61C 1/08, A61C 5/02, A61B 17/17, B23B 49/00

(54) **Stopper for cutting tool**
Anschlag für ein Schneidewerkzeug
Butoir pour outil coupant

(30) Priority: 26.07.2007 JP 2007193968
(43) Date of publication of application: 28.01.2009
(73) Proprietor: GC Corporation, Itabashi-ku Tokyo 174-8585 (JP)
(72) Inventor: Fujii, Naoto, Tokyo 174-8585 (JP); Takahashi, Masahi, Tokyo 174-8585 (JP); Mamada, Koichi, Tokyo 174-8585 (JP)
(74) Representative: Müller-Boré & Partner Patentanwälte

(56) References cited:
- US-A- 3 781 996
- US-A- 4 268 251
- US-A1- 2006 116 689

## Description

The present invention relates to a stopper for a cutting tool, which is used for a cutting tool such as a drill having a rotary shaft or a root canal cutting tool. The stopper is for restricting a cutting depth and making an idea of a cutting depth.

As for a conventional dental prosthesis in a defective tooth, a method using a bridge and a method using a base denture have been generally done. However, the method using a bridge includes the steps of cutting healthy natural teeth on both sides of a defective tooth to make abutment teeth, engaging a metal body with the abutment teeth, and fixing the metal body, where the metal body is attached to a dental prosthesis positioned at the defective tooth. Thus, engaging parts to make abutment teeth should be provided, where these abutment teeth are made by cutting healthy natural teeth. Further, since occlusal force ordinarily applied on a defective tooth is shared by abutment teeth, there is a problem that the life of natural teeth as abutment teeth is largely shortened. As for the method using a base denture, a dental prosthesis is made by fixing an artificial tooth made of a resin or ceramics on a base made of a synthetic resin. However, as for this method, the occlusal force applied on a dental prosthesis should be shared by remaining natural teeth and/or oral mucosae. Thus, there is an uncomfortable feeling while using a dental prosthesis, and taste of a patient becomes blunt because a denture base covers taste receptors dispersed in an oral mucosal tissue. Further, there are important fault that an alveolar ridge may be absorbed by using the denture base for a long period of time.

As for a treatment method to solve such faults, a dental implant technique is developed. The dental implant technique includes the steps of embedding an implant fixture as a maintaining and stabilizing device for a dental prosthesis into an embedding hole formed in a jawbone at a defective tooth, making the implant fixture to execute the function of a dental root in a natural teeth, connecting and fixing a fixing device for a dental prosthesis at the oral cavity inner side of the implant fixture to make a dental prosthesis maintaining device, and fixing a dental prosthesis to the dental prosthesis maintaining device.

In this implant treatment, it is important to correctly form a hole, in which a fixture is embedded, at a jawbone. Therefore, Patent Publication US 2005/0170311 A1, for example, discloses a method including the steps of taking a tomogram of a jawbone of a patent into a computer, producing a surgical template, in which an embedding direction of a fixture is previously determined and a hole specifying the embedding direction of the fixture is formed based on data correctly simulating complete states of a prosthesis including the fixture, attaching the surgical template into an oral cavity of a patient, and using a drill corresponding to the direction of the hole formed in the surgical template. At this time, a direction of the embedding hole is specified with the surgical template and depth of the embedding hole is specified with a flange shaped stopper integrally formed with a rotary shaft of a cutting drill.

However, it is necessary to use a flanged cutting tool in order to carry out the above-described treatment. Thus, many dentists who own a cutting tool having no stopper at a rotary shaft need to newly purchase a cutting tool corresponding to various kinds of cutting depths.

US 3, 781, 996 A, upon which prior art the two-part form of claim 1 is based, discloses an endodontic instrument for treating a pulp canal. The instrument includes an elongated member having a flated cutting portion at one end, a handle at the other and an intermediate part between the cutting portion and the handle. Selected stops of a set of reversible stop members are individually positionable on the intermediate portion. Each stop has an interior rubber holding section penetrated by the cutting portion to hold the stop.

US 4,268,251 A shows a boring needle for treatment of a dental root canal having a boring needle useful upon treating a dental root canal. The needle is secured at the proximal end to a grip member which is caused to stepwisely minutely slide relative to a sleeve member whereby a length of the needle projecting from the sleeve member can be finely adjusted by a determined minute distance.

Further, US 2006/0116689 A1 discloses a surgical instrument having an elongate member including a deformable distal portion having an initial configuration for placement within a spinal structure or other orthopedic structures, and a deformed configuration wherein the distal portion is outwardly deformed is provided.

An objective of the present invention is to provide a stopper for a cutting tool by which a treatment using a surgical template can be carried out with a conventional cutting tool having no a stopper at a rotary shaft.

Present inventors carried out earnest works to solve the above-described problems and, as a result, they found out the followings to complete the present invention. By supplying only a stopper separately from a cutting tool, a treatment using a surgical template can be carried with a conventional cutting tool having no stopper at a rotary shaft. Further, they noted that a surgical cutting tool is certainly heated and sterilized before using it. When a shape memory material which recovers a shape at a temperature for heating and sterilizing is used for a stopper for a cutting tool, the stopper for a cutting tool can be strongly fixed to a rotary shaft of the cutting tool after heating and sterilizing it.

That is, an aspect of the present invention is a stopper for a cutting tool to be fitted to a rotary shaft of a cutting tool having the rotary shaft. The stopper includes a locking member which has a through hole contacting to the rotary shaft of the cutting tool, and a clamping member which is made of a shape memory material, is provided at the inner and/or outer sides of the locking member and can deform the locking member by its shape recovering force. The stopper further includes a cutout part reaching to the through hole from an outer peripheral edge in the longitudinal direction of the rotary shaft.

A stopper for a cutting tool according to the present invention can make a conventional cutting tool having no stopper at a rotary shaft to carry out a treatment using a surgical template. Further, the stopper is an excellent one which hardly cause positional shift even when using a cutting drill or a dental root cutting tool.
Fig. 1 is a perspective view to illustrate a before heating state of one example of a stopper for a cutting tool according to the present invention.
Fig. 2 is a perspective view to illustrate one example of a cutting tool to which a stopper for a cutting tool is attached.
Fig. 3 is a perspective view to illustrate a state that the stopper for a cutting tool in Fig. 1 is temporarily attached to the cutting tool in Fig. 2.
Fig. 4 is a perspective view to illustrate a before heating state of another example of a stopper for a cutting tool according to the present invention.
Fig. 5 is a perspective view to illustrate an after heating state of further another example of a stopper for a cutting tool according to the present invention attached to the cutting tool in Fig. 2.

A stopper for a cutting tool according to the present invention will be described below with reference to the drawings.

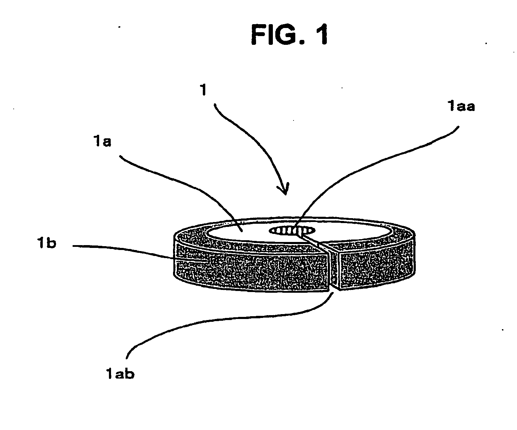

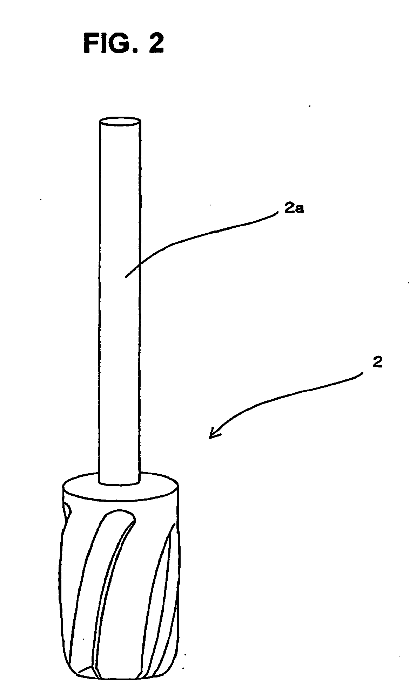

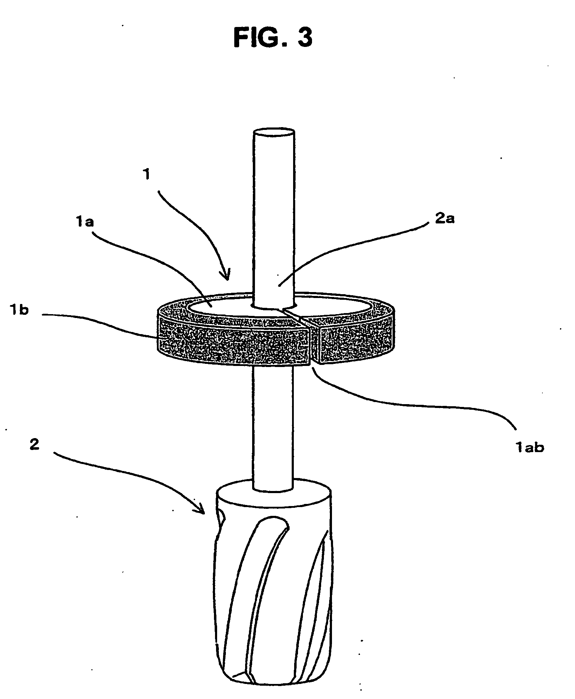

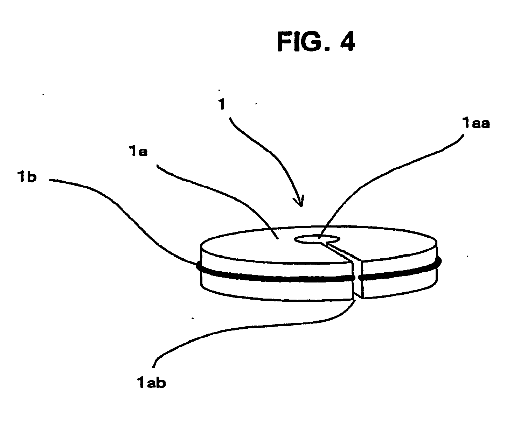

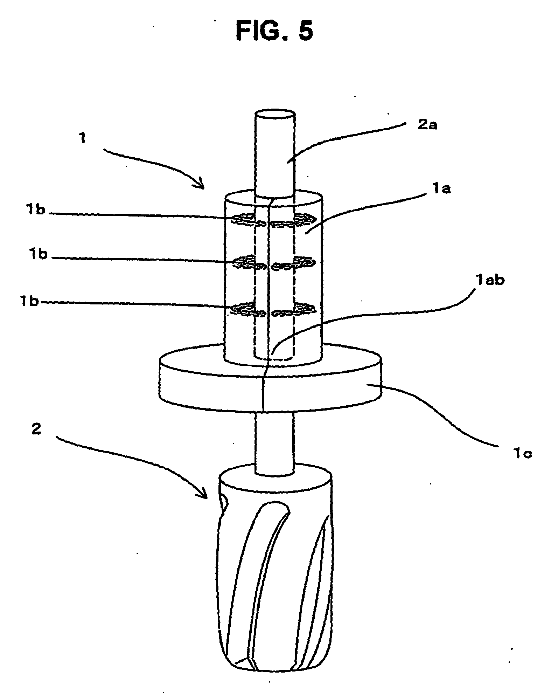

Fig. 1 is a perspective view to illustrate a before heating state of one example of a stopper for a cutting tool according to the present invention. Fig. 2 is a perspective view to illustrate one example of a cutting tool to which a stopper for a cutting tool is attached. Fig. 3 is a perspective view to illustrate a state that the stopper for a cutting tool in Fig. 1 is temporarily attached to the cutting tool in Fig. 2. Fig. 4 is a perspective view to illustrate a before heating state of another example of a stopper for a cutting tool according to the present invention. Fig. 5 is a perspective view to illustrate an after heating state of further another example of a stopper for a cutting tool according to the present invention attached to the cutting tool in Fig. 2.

A stopper 1 for a cutting tool according to the present invention in Fig. 1 is, for example, fitted to a rotary shaft 2a of a cutting tool 2 illustrated in Fig. 2 and used. The cutting tool 2 is not limited especially if having a drill shape and including a rotary shaft, and the cutting tool 2 may have or may not have a cutting edge at the rotary shaft.

The stopper 1 for a cutting tool according to the present invention includes a locking member 1a having a through hole 1aa contacting to the rotary shaft 2a, and includes a clamping member 1b, which is made of a shape memory material, provided at the inner and/or outer sides of the locking member 1a, and can deform the locking member 1a by its shape recovering force. The stopper 1 further includes a cutout part 1ab reaching to the through hole 1aa from an outer peripheral edge in the longitudinal direction of the rotary shaft 2a.

The locking member 1a is used so as to surround the rotary shaft 2a of the cutting tool 2. A shape of the locking member 1a is not limited especially if having the through hole 1aa contacting the rotary shaft 2a and having the cutout part 1ab. As for a disk-shaped stopper for a cutting tool illustrated in Figs, 1, 3 and 4, the through hole 1aa having a slightly larger diameter than an outer diameter of the rotary shaft 2a is formed in the locking member 1a.

The locking member 1a is fitted to the rotary shaft 2a by a clamping part 2 described below. As for the stopper 1 for a cutting tool according to the present invention, the clamping member 1b having a shape memory ability is recovered to clamp a rotary shaft by utilizing the heat at a temperature for heating and sterilizing (about 130°C). Thus, a material of the locking member 1a is not limited especially if surrounding the rotary shaft 2a of the cutting tool 2, for example, resin material or metal material. Preferably a flexible resin material having high thermal resistance. For example, this material includes polypropylene, polycarbonate, polyarylate, polysulfone, polyphenylene sulfide, polyether etherketone, a polyimide resin, and a fluororesin. Particularly, polyamide imide and polyether etherketone are preferable because of having thermal resistance at 250°C or more.

The clamping member 1b is made of a shape memory material. Plural numbers of the clamping members 1b may be provided inside the locking member 1a as illustrated in Fig. 5. The clamping member 1b may be fitted and fixed on the outer side of the locking member 1a so as to surround the locking member 1a as illustrated in Figs. 1 and 3. Further, the clamping member 1b may be fitted to a groove formed at an outer edge of the locking member 1 as illustrated in Fig. 4. In all cases, the clamping member 1b is provided so as to surround the rotary shaft 2a.

The clamping member 1b deforms the locking member 1a by its shape recovering force. The clamping member 1b makes the stopper 1 for a cutting tool fit to the rotary shaft 2 through the locking member 1, by being given a memory that a portion contacting a rotary shaft of the locking member 1a shrinks to be a smaller circle than the outer periphery of the rotary shaft and recovering the portion to have the shape of the memory by heating at the time of sterilizing. The clamping member 1b is preferably a shape memory alloy having a shape recovering temperature of about 50 to 100°C.

A stopper for a cutting tool according to the present invention includes a cutout part 1ab reaching to the through hole 1aa from an outer peripheral edge thereof in the longitudinal direction of the rotary shaft 2a of the cutting tool 2. The locking member 1a is deformed by the shape recovering force of the clamping member 1b so as to lose the cutout part 1ab, and then the stopper for a cutting tool is fitted to the rotary shaft 2a. The cutout part 1ab may be straight in the longitudinal direction of the rotary shaft 2a of the cutting tool 2 as illustrated Figs. 1, and 3 to 5, may be inclined with respect to the rotary shaft 2a, may be in a crank shape, or may be in a wavy shape (these states are not illustrated).

One example of a stopper for a cutting tool according to the present invention is in a disk shape as illustrated in Figs. 1 and 4. The stopper includes a through hole 1aa in a locking member 1a, and a cutout part 1ab reaching to the through hole 1aa. By this example, a cutting depth of a cutting tool can be correctly got. Another example is a stopper for a cutting tool which is formed in a washer shape or a coil shape as the whole with using a locking member to surround the rotary shaft 2a and using a shape memory material on the outer side of the locking member (this example is not illustrated).

Another example is a cylindrical stopper for a cutting tool including a flange part 1c at one end, a through hole 1aa, and a cutout part 1ab reaching to the through hole 1aa, as illustrated in Fig. 5. In this case, the stopper can be certainly fitted to the rotary shaft 2a of the cutting tool 2, and can get a correct cutting depth by the flange part 1c.

As for a using method of a stopper for a cutting tool according to the present invention, a stopper illustrated in Figs. 1, or 3 to 5 is used by the steps of previously expanding the cutout part 1ab so as to deform it to have a sufficiently larger shape than the outer periphery of the rotary shaft 2a in order to easily attach the through hole 1aa of the stopper 1 for a cutting tool to a predetermined position of the rotary shaft 2a, determining a depth of a cutting tool, deforming the cutout part 1ab by an operator (closing the cutout part by the easiest way), temporarily fixing the stopper for a cutting tool to the rotary shaft 2a by using a pliers or a specific tool, and carrying out a sterilizing treatment at a temperature of about 80 to 150°C in a heating and sterilizing apparatus such as an autoclave or a dryness oven according to an ordinary method before using the cutting tool. After the sterilizing treatment, since the stopper 1 for a cutting tool is certainly fitted to the rotary shaft 2a by the recovering force of the shape memory material 1b, the position of the stopper is not easily shifted at the time of using. After using the cutting tool, the stopper for a cutting tool is removed from the rotary shaft by deforming by a pliers or a specific tool, washed, and stored until the next time.

A stopper for a cutting tool according to the present invention can be used for a stopper conventionally used to restrict a cutting depth, e.g., a dental root canal cutting tool, in addition to a cutting drill for an implant.

## Claims

1. A stopper (1) for a cutting tool to be fitted to a rotary shaft (2a) of a cutting tool (2), the stopper (1) comprising:
a locking members (1a) which has a through hole (1aa) contacting to the rotary shaft (2a) of the cutting tool (2), **characterized in that** the stopper (1) further comprises
a clamping member (1b) which is made of a shape memory material, is provided at the inner and/or outer side of the locking member (1a) and can deform the locking member (1a) by its shape recovering force; and
a cutout part (lab) reaching to the through hole (1aa) from an outer peripheral edge of the stopper in the longitudinal direction of the rotary shaft (2a).

2. The stopper (1) for a cutting tool as claimed in claim 1, wherein the stopper (1) is in an approximately disk shape.

3. The stopper (1) for a cutting tool as claimed in claim 1, wherein the stopper (1) is in an approximately cylindrical shape having a flange (1c) at an end thereof.

## Patentansprüche

1. Stopper bzw. Anschlag (1) für ein Schneidwerkzeug, der an eine Drehwelle (2a) eines Schneidwerkzeugs (2) zu passen ist, wobei der Stopper (1) umfasst:
ein Verriegelungsglied (1a), das ein Durchgangsloch (1aa) aufweist, das die Drehwelle (2a) des Schneidwerkzeugs (2) kontaktiert, **dadurch gekennzeichnet, dass** der Stopper (1) ferner umfasst:
ein Klemmglied (1 b), das aus einem Formgedächtnismaterial besteht, an der inneren und/oder äußeren Seite des Verriegelungsglieds (1a) vorgesehen ist und das Verriegelungsglied (1a) durch seine Formerholungs- bzw. -wiedergewinnungskraft verformen kann; und
einen ausgeschnittenen Teil bzw. Ausschnittsteil (1ab), der von einem Außenumfangsrand bzw. -kante des Stoppers in der Längsrichtung der Drehwelle (2a) zu dem Durchgangsloch (1aa) reicht.

2. Stopper (1) für ein Schneidwerkzeug nach Anspruch 1, wobei der Stopper (1) näherungsweise die Form einer Scheibe aufweist.

3. Stopper (1) für ein Schneidwerkzeug nach Anspruch 1, wobei der Stopper (1) näherungsweise die Form eines Zylinders mit einem Flansch (1c) an einem Ende davon aufweist.

## Revendications

1. Une pièce d'arrêt (1) pour un outil coupant, à monter sur un axe rotatif (2a) d'un outil coupant (2), la pièce d'arrêt comprenant :
un élément de verrouillage (1a) qui a un trou débouchant (1aa) en contact avec l'axe rotatif (2a) de l'outil coupant (2), **caractérisée en ce que** la pièce d'arrêt (1) comprend en outre
un élément de serrage (1b) constitué d'un matériau à mémoire de forme est pourvu du côté interne et/ou externe de l'élément de verrouillage (1a) et peut déformer l'élément de verrouillage (1a) par sa force de récupération de forme ; et une partie découpée (1ab) s'étendant jusqu'au trou débouchant (1aa) depuis un bord périphérique extérieur de la pièce d'arrêt dans la direction longitudinale de l'axe rotatif (2a).

2. La pièce d'arrêt (1) pour un outil coupant selon la revendication 1, dans laquelle la pièce d'arrêt (1) a approximativement la forme d'un disque.

3. La pièce d'arrêt (1) pour un outil coupant selon la revendication 1, dans laquelle la pièce d'arrêt (1) a approximativement la forme d'un cylindre ayant une collerette (1c) à une de ses extrémités.
